# EUROPEAN PATENT APPLICATION

(11) **EP 4 303 878 A1**
(43) Date of publication of application: **10.01.2024**
(21) Application number: 22882454.6
(22) Date of filing: 31.08.2022
(51) Int. Cl.: G16C 20/30, G16B 15/10

(54) **METHOD FOR TRAINING MOLECULAR BINDING MODEL, MOLECULAR SCREENING METHOD AND APPARATUS, COMPUTER DEVICE, AND STORAGE MEDIUM**

(30) Priority: 19.10.2021 CN 202111213797
(71) Applicant: Tencent Technology (Shenzhen) Company Limited, Shenzhen, Guangdong, 518057 (CN)
(72) Inventor: BIAN, Yatao, Shenzhen, Guangdong 518057 (CN); XU, Tingyang, Shenzhen, Guangdong 518057 (CN)
(74) Representative: Gunzelmann, Rainer
(86) International application number: PCT/CN2022/116095
(87) International publication number: WO 2023/065838

(57) **Abstract**

The present disclosure provides a method for training a molecular binding model, and a molecular screening method and apparatus, which can be applied in the field of intelligent medicine, and used to solve the problem of low molecular screening accuracy. The screening method comprises: using a molecular binding model to be trained, determining binding activity feature information, embedded feature information and eutectic feature information between a sample protein molecule and a sample candidate molecule, on the basis of protein feature information and molecular feature information; on the basis of the binding activity feature information, the embedded feature information and the eutectic feature information, determining a training loss of the molecule binding model to be trained; if the training loss meets a training target, outputting the molecular binding model as a trained molecular binding model, the trained molecular binding model being used to determine binding activity feature information between a target protein molecule and a target candidate molecule, so as to predict binding activity of a compound after the target protein molecule and the target candidate molecule are virtually bound.

## Description

### RELATED APPLICATION

This application claims priority to Chinese Patent Application No. 202111213797.X, filed with the Chinese Patent Office on October 19, 2021, and entitled "METHODS FOR TRAINING MOLECULAR BINDING MODEL, METHODS FOR SCREENING MOLECULES AND APPARATUSES", the entire contents of which are incorporated by reference in this application.

### FIELD OF THE TECHNOLOGY

This application relates to the technical field of biological medicine, in particular to a method for training molecular binding model, a method for screening molecules, apparatuses, a computer device and a storage medium.

### BACKGROUND OF THE DISCLOSURE

As technology advances, more and more devices can use activity prediction models to predict the binding activity of compounds after virtual binding of molecules, so that some molecules can be screened for realistic drug binding experiments based on the predicted binding activity of the compounds.

When predicting the binding activity of the compounds after virtual binding of the molecules, the activity prediction model is usually based on the chemical property characteristics of the molecules extracted by a feature extraction model or the structural characteristics of the molecule itself to perform prediction.

### SUMMARY

Embodiments of this application provide a method for training a molecular binding model, a method for screening molecules, apparatuses, a computer device and a storage medium to be used for improving the accuracy for screening molecules.

An embodiment of this application provides a method for training molecular binding model, including:
obtaining protein feature information of a sample protein molecule and molecular feature information of a sample candidate molecule;
inputting the protein feature information and the molecular feature information into a molecular binding model, determining, using the molecular binding model, binding activity feature information, embedding feature information and eutectic feature information between the sample protein molecule and the sample candidate molecule, the binding activity feature information characterizing an activity of a compound produced by virtual binding of the sample protein molecule and the sample candidate molecule, the embedding feature information characterizing a degree of binding between the sample protein molecule and the sample candidate molecule, and the eutectic feature information characterizing whether a eutectic structure exists between the sample protein molecule and the sample candidate molecule;
determining a training loss of the molecular binding model based on the binding activity feature information, the embedding feature information and the eutectic feature information; and
outputting the molecular binding model as a trained molecular binding model in response to that the training loss of the molecular binding model meets a training target; the trained molecular binding model being configured to determine binding activity feature information between a target protein molecule and a target candidate molecule to predict a binding activity of a compound produced by virtual binding of the target protein molecule and the target candidate molecule.

An embodiment of this application provides a method for screening molecules, including:
obtaining a target protein molecule and a target candidate molecule;
extracting, using a feature extraction model, protein feature information of the target protein molecule and molecular feature information of the target candidate molecule;
determine, using a molecular binding model and based on the protein feature information and the molecular feature information, binding activity feature information between the target protein molecule and the target candidate molecule; the molecular binding model being trained and obtained based on the method for training molecular binding model described above; and
predict, using an activity prediction model and based on the binding activity feature information, an activity value of a compound produced by virtual binding of the target protein molecule and the target candidate molecule.

An embodiment of this application provides an apparatus for training a molecular binding model, including:
an obtaining module, configured to obtain protein feature information of a sample protein molecule and molecular feature information of a sample candidate molecule; and
a processing module, configured to input the protein feature information and the molecular feature information into a molecular binding model, and determine, using the molecular binding model, binding activity feature information, embedding feature information and eutectic feature information between the sample protein molecule and the sample candidate molecule, the binding activity feature information characterizing an activity of a compound produced by virtual binding of the sample protein molecule and the sample candidate molecule, the embedding feature information characterizing a degree of binding between the sample protein molecule and the sample candidate molecule, and the eutectic feature information characterizing whether a eutectic structure exists between the sample protein molecule and the sample candidate molecule;
determine a training loss of the molecular binding model based on the binding activity feature information, the embedding feature information and the eutectic feature information; and
output the molecular binding model as a trained molecular binding model in response to that the training loss of the molecular binding model meets a training target; the trained molecular binding model being configured to determine binding activity feature information between a target protein molecule and a target candidate molecule to predict a binding activity of a compound produced by virtual binding of the target protein molecule and the target candidate molecule.

An embodiment of this application further provides an apparatus for screening molecules, including:
an obtaining module, configured to obtain a target protein molecule and a target candidate molecule;
a processing module, configured to extract, using a feature extraction model, protein feature information of the target protein molecule and molecular feature information of the target candidate molecule;
the processing module being further configured to: determine, using a molecular binding model and based on the protein feature information and the molecular feature information, binding activity feature information between the target protein molecule and the target candidate molecule; the molecular binding model being trained and obtained based on the method for training molecular binding model described above;
the processing module being further configured to: predict, using an activity prediction model and based on the binding activity feature information, an activity value of a compound produced by virtual binding of the target protein molecule and the target candidate molecule.

An embodiment of this application further provides a computer device, including:
a memory, configured to store program instructions; and
a processor, configured to call the program instructions stored in the memory, to execute, in accordance with the obtained program instructions, the method for training molecular binding model or the method for screening molecules described above.

An embodiment of this application provides a computer-readable storage medium, the computer storage medium storing computer-executable instructions, the computer-executable instructions being used for enabling a computer to execute the method for training molecular binding model or the method for screening molecules described above.

An embodiment of this application further provides a computer program product, the computer program product including computer instructions, the computer instructions being stored in a computer-readable storage medium, the computer instructions, when being executed by the computer instructions, implementing the method for training molecular binding model or the method for screening molecules described above.

### BRIEF DESCRIPTION OF THE DRAWINGS

FIG. 1 is a schematic diagram of a principle of a method for training molecular binding model according to an embodiment of this application;
FIG. 2 shows an application scenario of the method for training molecular binding model according to the embodiment of this application;
FIG. 3 is a schematic flowchart of the method for training molecular binding model according to the embodiment of this application;
FIG. 4a is a structural schematic diagram of molecules of the method for training molecular binding model according to the embodiment of this application;
FIG. 4b is a structural schematic diagram of another molecules of the method for training molecular binding model according to the embodiment of this application;
FIG. 4c is a schematic diagram of another principle of the method for training molecular binding model according to the embodiment of this application;
FIG. 4d is a schematic diagram of another principle of the method for training molecular binding model according to the embodiment of this application;
FIG. 4e is a schematic diagram of another principle of the method for training molecular binding model according to the embodiment of this application;
FIG. 5a is another schematic flowchart of the method for training molecular binding model according to the embodiment of this application;
FIG. 5b is a structural schematic diagram of another molecules of the method for training molecular binding model according to the embodiment of this application;
FIG. 5c is a structural schematic diagram of another molecules of the method for training molecular binding model according to the embodiment of this application;
FIG. 5d is a structural schematic diagram of another molecules of the method for training molecular binding model according to the embodiment of this application;
FIG. 5e is a structural schematic diagram of another molecules of the method for training molecular binding model according to the embodiment of this application;
FIG. 6 is a schematic diagram of another principle of the method for training molecular binding model according to the embodiment of this application;
FIG. 7 is another schematic flowchart of a method for screening molecules according to an embodiment of this application;
FIG. 8 is another structural schematic diagram of an apparatus for training molecular binding model according to an embodiment of this application;
FIG. 9 is another structural schematic diagram of an apparatus for screening molecules according to an embodiment of this application; and
FIG. 10 is a structural schematic diagram of the apparatus for training molecular binding model or the apparatus for screening molecules according to the embodiment of this application.

### DESCRIPTION OF EMBODIMENTS

To make the objectives, technical solutions, and advantages of the embodiments of this application clearer, the following clearly and completely describes the technical solutions in the embodiments of this application with reference to the accompanying drawings in the embodiments of this application.

Some of the terms used in the embodiments of this application are explained below to facilitate understanding by those skilled in the art.

### (1) 50% inhibitory concentrations IC50 and pIC50:

IC50 may also be referred to as the 50% inhibition rate, and the concentration of drug or inhibitor required to inhibit half of a specified biological process. There is a mathematical relationship between pIC50 and IC50, with pIC50 being the opposite number of the logarithm of IC50.

### (2) Protein molecules and molecules:

Protein molecules are the material basis of life, organic macromolecules, the basic organic substances that make up cells and the main bearers of vital activities, and amino acid molecules are the basic constituent units of the protein molecules.

Molecules are composed of atoms which are bound together in a certain bonding order and spatial arrangement. The bonding order and spatial arrangement relationship are the molecular structures, which can also be referred to as small molecules in the embodiments of this application.

### (3) Virtual screening

In the process of drug research and development, drug screening is one of the main ways to discover lead compounds. Physical drug screening requires the construction of a large-size compound library, the extraction or cultivation of a large number of experimentally necessary target enzymes or target cells, and the support of complex devices, so physical drug screening requires a huge capital investment. Virtual drug screening allows for preliminary screening of drug compounds prior to physical screening, thus significantly saving the cost.

In the embodiments of this application, candidate molecules refer to candidate molecules for virtual drug screening.

The embodiments of this application relate to the Artificial Intelligence (AI) technology. The design is based on the Computer Vision (CV) technology and the Machine Learning (ML) technology in the AI technology.

The following is a brief description of the fields of application of methods for training molecular binding model according to an embodiment of this application.

As technology advances, more and more devices can use activity prediction model to predict the binding activity of compounds after virtual binding of molecules, so that some molecules can be screened for realistic drug binding experiments based on the predicted binding activity of the compounds, avoiding the situation where some unnecessary drug binding experiments are performed, resulting in a waste of resources.

When predicting the binding activity of the compounds after virtual binding of the molecules, the activity prediction model is usually based on the chemical property characteristics of the molecules extracted by a feature extraction model or the structural characteristics of the molecule itself to perform prediction on the binding activity between two molecules.

However, in the molecular binding process, the two molecules are not simply put together, and there may be unpredictable interactions between the molecules. Only from the perspective of the molecules themselves, the prediction of the binding activity of a compound after virtual binding of two molecules is less accurate, resulting in low accuracy for screening molecules. As a result, some molecules that are necessary for binding are not screened out for drug binding experiments, while some molecules that are not necessary for binding are screened out for drug binding experiments, which will not only cause waste of resources, but also affect the progress of drug research and development.

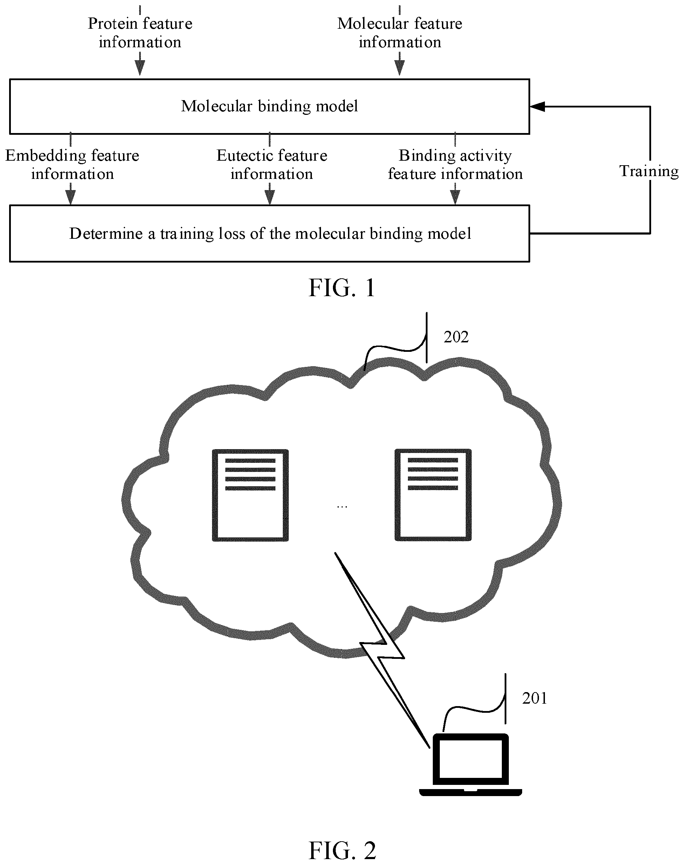

In order to solve the problem that the accuracy for screening molecules is low, an embodiment of this application provides a method for training molecular binding model. Please refer to FIG. 1, in this method, respective protein feature information of each sample protein molecule and respective molecular feature information of each sample candidate molecule are obtained. Based on each protein feature information and each molecular feature information, a molecular binding model is iteratively trained for many times until a training loss of the molecular binding model meets a training target, and the trained molecular binding model is output, the trained molecular binding model being configured to determine binding activity feature information of two molecules (e.g., a target protein molecule and a target candidate molecule) after virtual binding to predict the binding activity of a compound after virtual binding of the two molecules, thereby performing virtual drug screening.

For iterative training with many times, the following operations are performed respectively: use the molecular binding model to determine, based on the protein feature information and the molecular feature information, the binding activity feature information, embedding feature information and eutectic feature information between the sample protein molecule and the sample candidate molecule, the binding activity feature information characterizing the activity of the sample protein molecule and the sample candidate molecule after virtual binding, the embedding feature information characterizing the degree of binding between the sample protein molecule and the sample candidate molecule, the eutectic feature information characterizing whether a eutectic structure exists between the sample protein molecule and the sample candidate molecule, and the molecular binding model being configured to, based on the binding activity feature information, the embedding feature information and the eutectic feature information, determine the training loss of the molecular binding model.

In the embodiments of this application including the embodiments of both the claims and the specification (hereinafter referred to as "all embodiments of the present disclosure"), after obtaining the respective protein feature information of each sample protein molecule and the respective molecular feature information of each sample candidate molecule, the binding activity feature information, the embedding feature information and the eutectic feature information between the sample protein molecule and the sample candidate molecule are further obtained based on each protein feature information and each molecular feature information; based on the binding activity feature information, the embedding feature information and the eutectic feature information, the molecular binding model is trained; the molecular binding model is trained from the perspective of multiple feature information, so that when predicting the binding activity of molecules after virtual binding, an activity prediction model takes into account not only the chemical property characteristics of molecules contained in the protein feature information and the molecular feature information, or the structural characteristics of the molecules themselves, but also the intermolecular interactions such as intermolecular embedding characteristics and eutectic characteristics. This makes the activity value predicted by the activity prediction model based on the molecular binding model more accurate.

The following illustrates the application scenario of the method for training molecular binding model according to this application.

Please refer to FIG. 2, which shows an application scenario of the method for training molecular binding model according to the embodiment of this application. The application scenario includes a client 101 and a server 102. Communication may be performed between the client 101 and the server 102 and may be performed by means of a wired communication technology, for example by connecting a network cable or a serial port cable; communication may also be performed by means of a wireless communication technology, for example by means of Bluetooth or wireless fidelity (WIFI) etc., which is not specifically limited.

The client 101 generally refers to a device that can instruct the server 102 to train molecular binding model or perform molecular screening, for example, a terminal device, a third-party application accessible by the terminal device or a web page accessible by the terminal device, etc. The terminal device includes, but is not limited to, a mobile phone, a computer, a smart medical device, a smart electric appliance, etc. The server 102 refers generally to a device that can train molecular binding model or perform molecular screening, for example, a terminal device or a server, etc. The server includes, but is not limited to, a cloud server, a local server or an associated third-party server, etc. Both the client 101 and the server 102 may use cloud computing so as to reduce the occupation of local computing resources; cloud storage may likewise be used to reduce the occupation of local storage resources.

As an embodiment, the client 101 and the server 102 may be the same device, and in the embodiment of this application, the client 101 and the server 102 are introduced as different devices as an example.

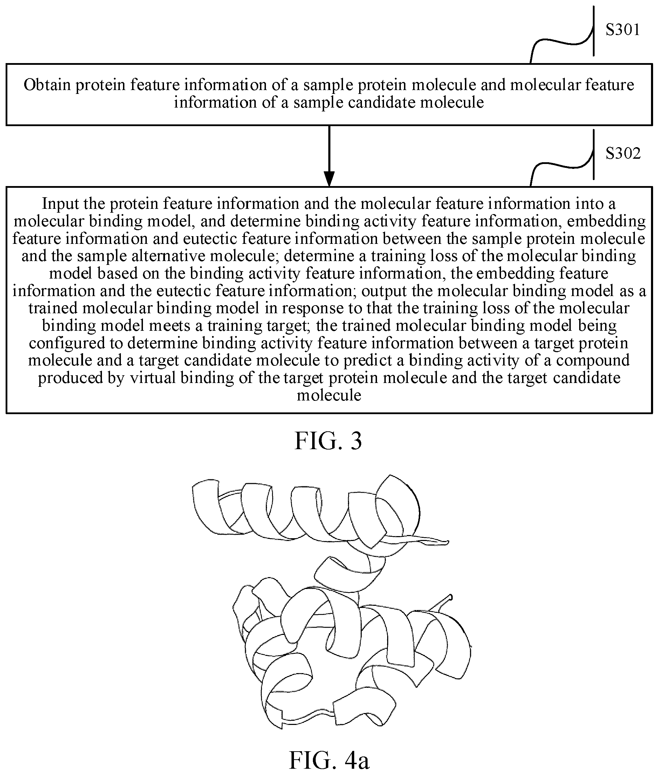

Based on FIG. 3, the method for training molecular binding model according to the embodiment of this application is specifically introduced below, with the client 101 as a target client and the server 102 as a server as an example.

Please refer to FIG. 3, which is a schematic flowchart of the method for training molecular binding model according to the embodiment of this application.

S301: Obtain protein feature information of a sample protein molecule and molecular feature information of a sample candidate molecule.

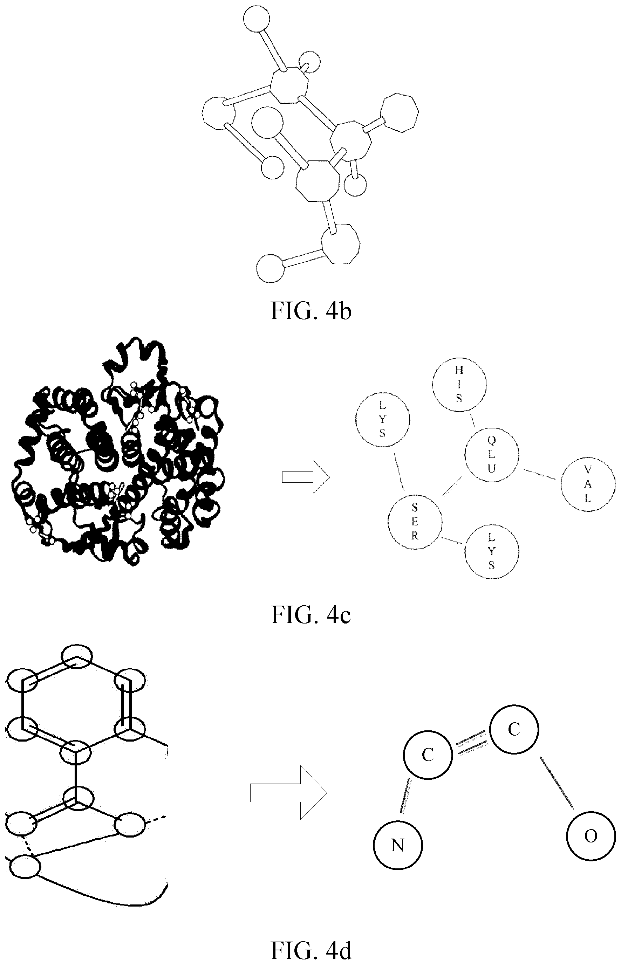

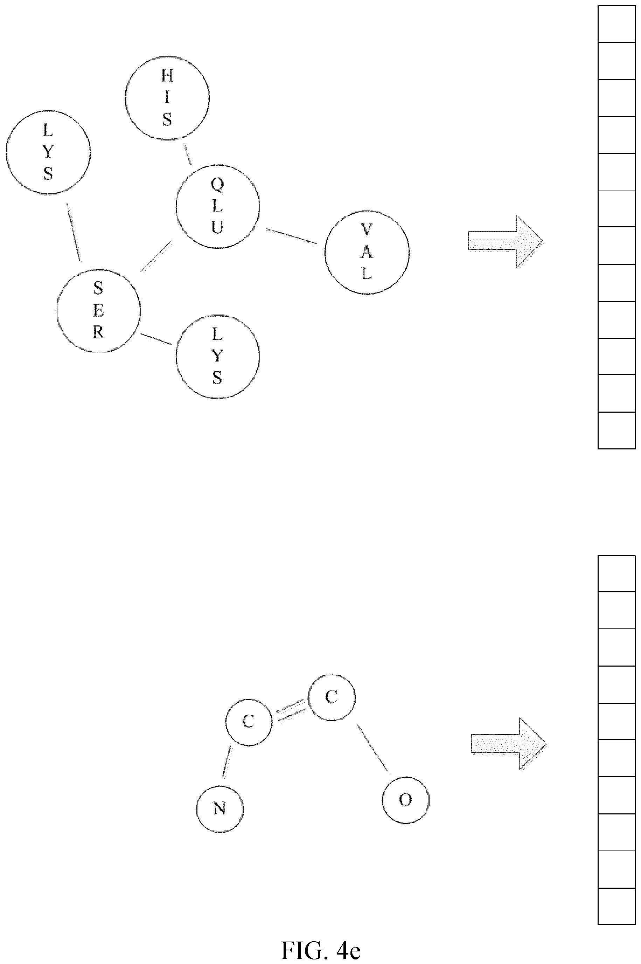

The server may receive the protein feature information of the sample protein molecule and the molecular feature information of the sample candidate molecule sent by other devices, and may also perform feature extraction processing on the sample protein molecule and the sample candidate molecule separately to obtain the protein feature information of the sample protein molecule and the molecular feature information of the sample candidate molecule, etc., which is not specifically limited. For example, FIG. 4a illustrates an exemplary structural schematic diagram of a sample protein molecule. FIG. 4billustrates an exemplary structural schematic diagram of a sample candidate molecule.

In all embodiments of the present disclosure, the topological structure of the sample protein molecule and the sample candidate molecule can be effectively introduced by transforming the structural information of the sample protein molecule and the sample candidate molecule into an adjacency matrix as an input of a molecular binding model. The topological structure of a compound can influence the features of the whole compound, so the molecular binding model obtains the feature information of the sample protein molecule and the sample candidate molecule by means of the topological structure of the sample protein molecule and the sample candidate molecule, such as a feature matrix representation.

The embodiment of this application is introduced by taking that a server uses a feature extraction model to extract the protein feature information of the sample protein molecule and the molecular feature information of the sample candidate molecule as an example.

In all embodiments of the present disclosure, the obtain protein feature information of a sample protein molecule and molecular feature information of a sample candidate molecule may include:
obtain training samples, the training sample including the sample protein molecule and the sample candidate molecule; and
use a feature extraction model to perform feature extraction processing on the training sample to obtain protein feature information of the sample protein molecule and molecular feature information of the sample candidate molecule.

In all embodiments of the present disclosure, the server may first obtain a training sample set, the training sample set including a plurality of training samples, each training sample including a sample protein molecule and a sample candidate molecule.

The server may extract, based on a database, a plurality of sample protein molecules and a plurality of sample candidate molecules which have undergone drug binding experiments to obtain the training sample set; the plurality of sample protein molecules and the plurality of sample candidate molecules which have undergone drug binding experiments may also be found in web resources, thereby obtaining the training sample set, with no specific limitations on a method for obtaining training sample set.

After obtaining the training sample set, the server can use the feature extraction model to perform feature extraction processing on each training sample to obtain protein feature information of the sample protein molecule and molecular feature information of the sample candidate molecule in each training sample. The feature extraction model is configured to perform feature extraction processing on the molecule to obtain the structural information of the molecule. A feature extraction model server may input respective three-dimensional structural diagrams of the sample protein molecule and the sample candidate molecule into the feature extraction model to perform feature extraction processing on each training sample respectively; may also input respective multi-perspective two-dimensional planar structural diagrams of the sample protein molecule and the sample candidate molecule into the feature extraction model to perform feature extraction processing, etc. on each training sample, without specific limitation.

As an embodiment, when the server uses the feature extraction model to perform feature extraction processing on each training sample respectively, an adjacency matrix may be used for performing calculation to introduce the process of performing feature extraction processing on one training sample, and the process of performing feature extraction processing on other training samples is similar and will not be repeated here.

The server determines an adjacency matrix of the sample protein molecule based on the sample protein molecule in the training sample; please refer to FIG. 4c, the adjacency matrix of the sample protein molecule is used for characterizing various sample amino acid molecules contained in the sample protein molecule and the molecular structure distance between every two sample amino acid molecules. In response to determining the adjacency matrix of the sample protein molecule, the server may determine the adjacency matrix of the sample protein molecule based on the various sample amino acid molecules contained in the sample protein molecule, and the molecular structure distance between every two sample amino acid molecules, may also build the adjacency matrix of the sample protein molecule based on the various sample amino acid molecules, and the molecular structure distances greater than a preset distance threshold, and may also determine, using the various sample amino acid molecules as vertices, after determining the molecular structure distance between every two sample amino acid molecules in the sample protein molecule, whether the various molecular structure distances are greater than a preset distance threshold, and establish an edge between corresponding two sample amino acid molecules with a molecular structure distance greater than the preset distance threshold, so that a relationship graph between corresponding vertices of the sample protein molecule and edges between the vertices can be obtained. The server builds, based on the obtained relationship graph, the adjacency matrix of the sample protein molecule, etc., without any specific limitation.

The server determines an adjacency matrix of the sample candidate molecule based on the sample candidate molecule in the training sample; please refer to FIG. 4d, the adjacency matrix of the sample candidate molecule characterizes various sample candidate atoms contained in the sample candidate molecule, and chemical bond structures between the various sample candidate atoms. In response to determining the adjacency matrix of the sample candidate molecule, the server may determine the adjacency matrix of the sample candidate molecule based on the various sample candidate atoms contained in the sample candidate molecule, and the chemical bond structure between the various sample candidate atoms, may also build the adjacency matrix of the sample candidate molecule based on the various sample candidate atoms, and chemical bonds between the sample candidate atoms in the chemical bond structure, and may also obtain a relationship graph of corresponding vertices of the sample candidate atoms and edges between the vertices, with the various sample candidate atoms as the vertices and the chemical bonds between the sample candidate atoms as edges. Based on the obtained relationship graph, the server builds the adjacency matrix of the sample candidate molecule, without any specific limitations.

The server performs feature extraction processing on the adjacency matrix of the sample protein molecule and the adjacency matrix of the sample candidate molecule, respectively; please refer to FIG. 4e, the protein feature information of the sample protein molecule, and the molecular feature information of the sample candidate molecule are obtained.

S302: Input the protein feature information and the molecular feature information into a molecular binding model, determine, using the molecular binding model, binding activity feature information, embedding feature information and eutectic feature information between the sample protein molecule and the sample candidate molecule; determine a training loss of the molecular binding model based on the binding activity feature information, the embedding feature information and the eutectic feature information; and output the molecular binding model as a trained molecular binding model in response to that the training loss of the molecular binding model meets a training target; the trained molecular binding model being configured to determine binding activity feature information between a target protein molecule and a target candidate molecule to predict a binding activity of a compound produced by virtual binding of the target protein molecule and the target candidate molecule.

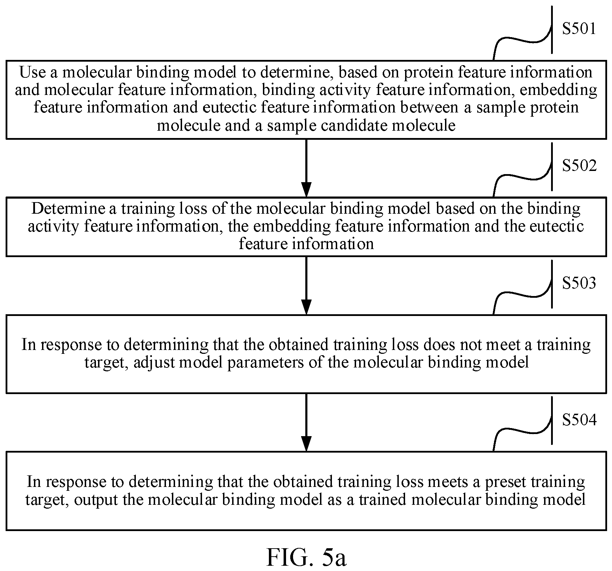

After obtaining the protein feature information of the sample protein molecule and the molecular feature information of the sample candidate molecule, the server may perform iterative training for many times on the molecular binding model based on the protein feature information and the molecular feature information, the process of each iterative training is similar, the process of one iterative training is introduced below, and please refer to FIG. 5a.

S501: determine, using a molecular binding model and based on the protein feature information and the molecular feature information, the binding activity feature information, the embedding feature information and the eutectic feature information between the sample protein molecule and the sample candidate molecule.

After obtaining the protein feature information and the molecular feature information, the server can use the molecular binding model to predict the binding activity feature information between the sample protein molecule and the sample candidate molecule, the binding activity feature information being configured to predict the binding activity feature of the sample protein molecule and the sample candidate molecule after virtual binding. At the same time, the server can also use the molecular binding model to predict the embedding feature information and the eutectic feature information between the sample protein molecule and the sample candidate molecule, and train the molecular binding model based on the embedding feature information and the eutectic feature information, so that the molecular binding model can have the performance of identifying the embedding relationship and the eutectic structure between molecules after virtual binding, so that when the binding activity feature information between molecules after virtual binding is predicted, more accurate binding activity feature information can be predicted, and when the activity value is predicted by the activity prediction model based on the binding activity feature information obtained by the molecular binding model, more accurate binding activity can be obtained .

The process of predicting the embedding feature information and the eutectic feature information between the sample protein molecule and the sample candidate molecule using the molecular binding model is described below.

The server can predict, based on the protein feature information and the molecular feature information, the binding distance between the various sample amino acid molecules contained in the sample protein molecule, and the various sample candidate atoms contained in the sample candidate molecule, after virtual binding of the sample protein molecule and the sample candidate molecule. In all embodiments of the present disclosure, the binding distance may be a distance between each sample amino acid molecule and each sample candidate atom calculated from the protein feature information and the molecular feature information. The server may determine the embedding feature information and the eutectic feature information between the sample protein molecule and the sample candidate molecule based on the various binding distances obtained.

As an embodiment, a residue of a specified sample amino acid molecule can bind to a cavity of an sample candidate atom to form an embedding relationship; therefore, the server can determine the embedding feature information based on various binding distances, and then introduce supervision information for the molecular binding model by means of graph matching, so that the trained molecular binding model has the performance of identifying the embedding feature between molecules, and the accuracy for predicting, based on the trained molecular binding model, the binding activity between molecules after virtual binding is improved.

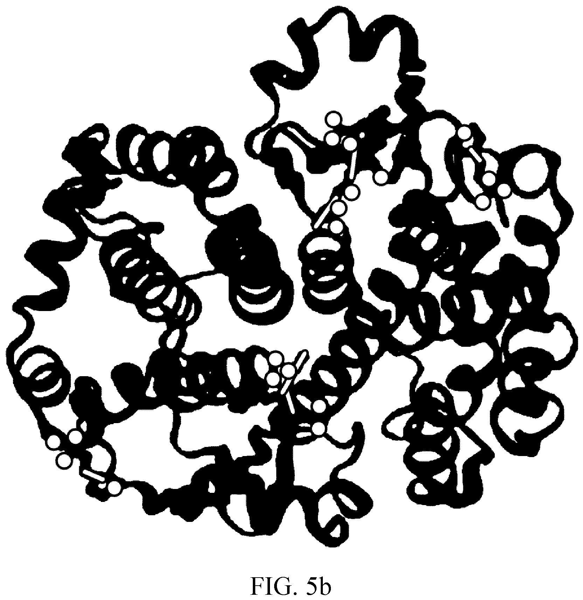

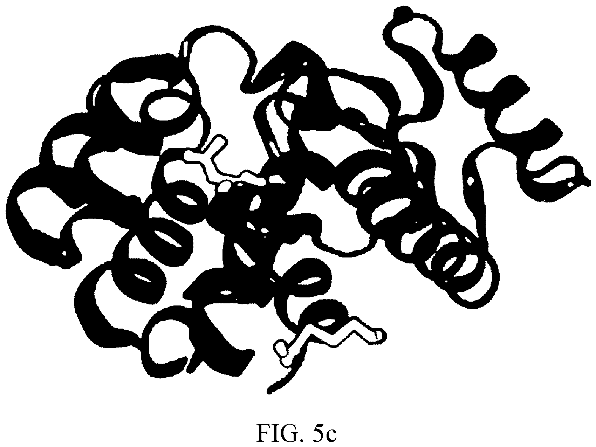

After obtaining the binding distance between each sample amino acid molecule contained in the sample protein molecule and each sample candidate atom contained in the sample candidate molecule, the server can determine, based on the binding distance with a minimum value between the specified sample amino acid molecule and each sample candidate atom, the embedding feature information between the sample protein molecule and the sample candidate molecule. The specified sample amino acid molecule may be a sample amino acid molecule having a targeting property in the sample protein molecule, or a sample amino acid molecule specified according to a scenario, etc., without specific limitation. Training is stabilized to a minimum value by means of the binding distance with a minimum value between the specified sample amino acid molecule and each sample candidate atom such that the residue of the sample amino acid molecule binds to the cavity of the corresponding sample candidate atom to form an embedding relationship. Please refer to FIG. 5b, which is a structural schematic diagram of the embedding relationship, with the sample candidate molecule fully embedded in the sample protein molecule. Please refer to FIG. 5c, which is a structural schematic diagram of the embedding relationship, with the sample candidate molecule partially embedded in the sample protein molecule.

As an embodiment, in response to that the sample amino acid molecule matches the sample candidate atom, then there is a eutectic structure between the sample amino acid molecule and the sample candidate atom, i.e., the eutectic structure is reflected in the matching of the sample candidate atom and the sample amino acid, therefore the server can determine the eutectic feature information based on the various binding distances and then introduce supervision information for the molecular binding model by means of neighborhood consensus, so that the trained molecular binding model has the performance of identifying intermolecular eutectic features and the accuracy of predicting, based on the trained molecular binding model, the binding activity between molecules after virtual binding is improved.

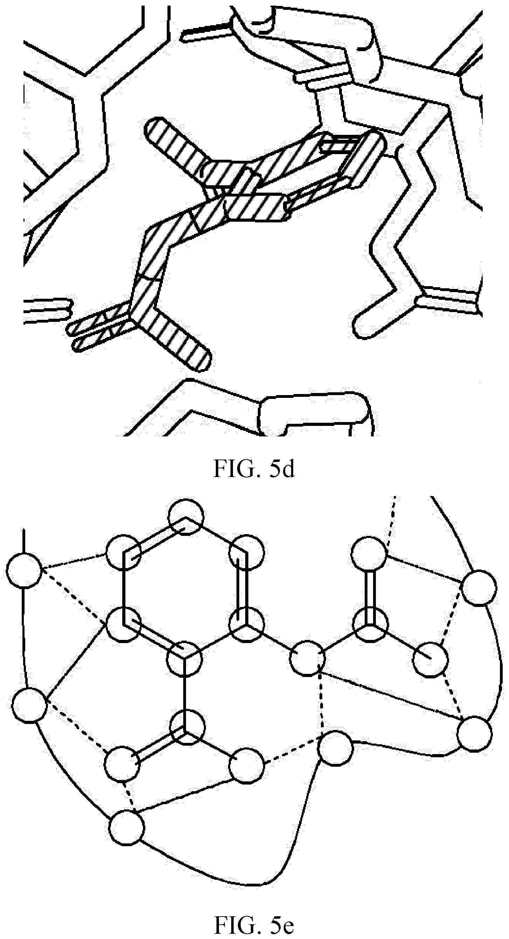

After obtaining the binding distance between each sample amino acid molecule contained in the sample protein molecule and each sample candidate atom contained in the sample candidate molecule, the server can determine, based on each sample amino acid molecule, each sample candidate atom, and a binding distance between each sample amino acid molecule and the sample candidate atom, the eutectic feature information between the sample protein molecule and the sample candidate molecule, where the eutectic feature information can be represented in the form of an adjacency matrix. Training is stabilized to be close to a eutectic target by means of the binding distance between the sample candidate atom and the sample amino acid molecule which are matched such that between the sample candidate atom and the sample amino acid molecule which are matched, a eutectic structure is formed; please refer to FIG. 5d, which is a structural schematic diagram of the eutectic structure; please refer to FIG. 5e, which is another structural schematic diagram of the eutectic structure.

S502: Determine the training loss of the molecular binding model based on the binding activity feature information, the embedding feature information and the eutectic feature information.

After obtaining the binding activity feature information, the embedding feature information and the eutectic feature information, the server may determine the training loss of the molecular binding model based on the binding activity feature information, the embedding feature information and the eutectic feature information, and train the molecular binding model based on the training loss.

The server may perform weighted fusion processing on the binding activity feature information, the embedding feature information and the eutectic feature information to obtain fused feature information, and determine the training loss of the molecular binding model by means of an error value between the fused feature information and the training target.

The server may also determine a first training loss of the molecular binding model based on a first error value between the embedding feature information and an embedding target, determine a second training loss of the molecular binding model based on a second error value between the eutectic feature information and a eutectic target, determine a third training loss of the molecular binding model based on a third error value between the binding activity feature information and an activity target, and determine the training loss of the molecular binding model based on the first training loss, the second training loss and the third training loss which are obtained. The server may determine the training loss of the molecular binding model based on the weighted sum of the first training loss, the second training loss and the third training loss, and may also directly determine the first training loss, the second training loss and the third training loss as the training loss of the molecular binding model, etc.

As an embodiment, the server may determine, by means of a graph matching model, a first error value between the binding distance, characterized by the embedding feature information, between the specified sample amino acid molecule and the corresponding sample candidate atom, and a reference binding distance. The server determines, based on the first error value, the first training loss of the molecular binding model. The reference binding distance may be obtained from a PDBbind dataset.

As an embodiment, it is easier to train a neighborhood consensus model as it has a more single function and more training data, and the accuracy of a matching matrix obtained using the trained neighborhood consensus model is already at a high level. At this moment, the trained neighborhood consensus model can be used to determine a eutectic target. The server can use the trained neighborhood consensus model to match each sample amino acid molecule contained in the sample protein molecule with each sample candidate atom contained in the sample candidate molecule to obtain matching feature information between the sample protein molecule and the sample candidate molecule as the eutectic target, the matching feature information being used for characterizing the matching distance between each sample amino acid molecule and each sample candidate atom. The server may use a cross entropy function to determine a second error value between the eutectic feature information and the eutectic target, and uses the second error value as the second training loss of the molecular binding model. The trained neighborhood consensus model may be trained and obtained based on data in the PDBbind dataset, and the cross entropy function may be replaced with other functions, without specific limitation.

As an embodiment, in the various training samples contained in the training sample set, in addition to the sample protein molecule and the sample candidate molecule, a reference activity value of the sample protein molecule and the sample candidate molecule after virtual binding is further included, the reference activity value being used for characterizing the true activity of the sample protein molecule and the sample candidate molecule after the drug binding experiment. If the sample protein molecule and the sample candidate molecule are obtained based on the drug binding experiment, the reference activity value is obtained based on the experimental results of the drug binding experiment of the sample protein molecule and the sample candidate molecule. If the sample protein molecule and the sample candidate molecule are obtained from a web resource, then the reference activity value is also obtained from the corresponding web resource, etc., without specific limitation. The training sample set can be obtained from the PDBbind dataset.

The server can use the activity prediction model to predict, based on the binding activity feature information, the sample activity value after virtual binding of the sample protein molecule and the sample candidate molecule, and determine, based on the error value between the sample activity value and a corresponding reference activity value, the third training loss of the molecular binding model.

In all embodiments of the present disclosure, prediction of the sample activity value may include: for a pair of given sample protein molecule and sample candidate molecule, the process of abstracting the sample protein molecule into a graph takes amino acids as vertices, uses the pdb structure file of the protein, takes the protein side chain heavy atom CB (CA is selected if CB is not present) therein as the center of the amino acid, calculates the Euclidean distance between the amino acid centers, and then by some normalized processing, obtains an adjacency matrix representing the protein structure. For the sample candidate molecule, the adjacency matrix thereof is also obtained in the same way by using atoms directly as vertices. Two graphs are then input into corresponding Encoders to obtain vector representation matrices of the sample protein molecule and the sample candidate molecule respectively, and the two vector representation matrices obtained are subjected to Graph Matching and Neighborhood Consensus as described above, while prediction of the sample activity value is obtained for the vector representation matrices of the sample protein molecule and the sample candidate molecule through an output layer of a neural network.

The server can determine whether the first training loss meets a first training target, the second training loss meets a second training target, and the third training loss meets a third training target, respectively. The training loss of the molecular binding model is determined to meet the training target when the first training loss, the second training loss and the third training loss all meet the corresponding training targets.

As an embodiment, the training target may be a preset target error value or a convergence state, without specific limitation.

After obtaining the first training loss, the second training loss and the third training loss, it can be determined whether the first training loss converges, whether the second training loss converges, and whether the third training loss converges, respectively, thereby determining whether the training loss of the molecular binding model meets the training target. In the event that the first training loss, the second training loss and the third training loss all converge, it is determined that the training loss of the molecular binding model meets the training target.

S503: In response to determining that the obtained training loss does not meet the training target, adjust model parameters of the molecular binding model.

If the obtained training loss does not meet the training target, it means that the predictive ability of the molecular binding model is not accurate enough and further training is needed and current model parameters need to be further adjusted, then the model parameters of the molecular binding model are adjusted.

As an embodiment, in response to that at least one of the first training loss, the second training loss and the third training loss does not converge, then it is determined that the obtained training loss does not meet the training target and the model parameters of the molecular binding model are adjusted.

S504: In response to determining that the obtained training loss meets the preset training target, output the molecular binding model as a trained molecular binding model.

If the obtained training loss meets the training target, it means that the predictive ability of the molecular binding model is already more accurate and current model parameters can no longer be adjusted, then the molecular binding model is output as the trained molecular binding model.

As an embodiment, the molecular binding model may be trained in a gradient manner, and the binding activity information may contain pIC50.

The training process in the embodiment of this application is implemented in an end-to-end format, without the need for artificial sub-problem partitioning, but is delivered entirely to the model to learn the mapping from original data to desired output directly, improving the intelligence for training the model and avoiding the problem that due to unpredictable circumstances in human involvement, the prediction accuracy of the trained model is low.

After obtaining the trained molecular structure model, the server can combine the feature extraction model, a target molecular structure model and the activity prediction model to predict the binding activity of the target protein molecule and the target candidate molecule after virtual binding. After obtaining the target protein molecule and the target candidate molecule, the server uses the feature extraction model to extract the feature of the target protein molecule and the target candidate molecule respectively, and obtains the protein feature information and the molecular feature information. Based on the protein feature information and the molecular feature information, the server uses the trained molecular structure model to obtain the binding activity feature information of the target protein molecule and the target candidate molecule after virtual binding. Based on the binding activity feature information, the server uses the activity prediction model to predict the activity value of the target protein molecule and the target candidate molecule after virtual binding.

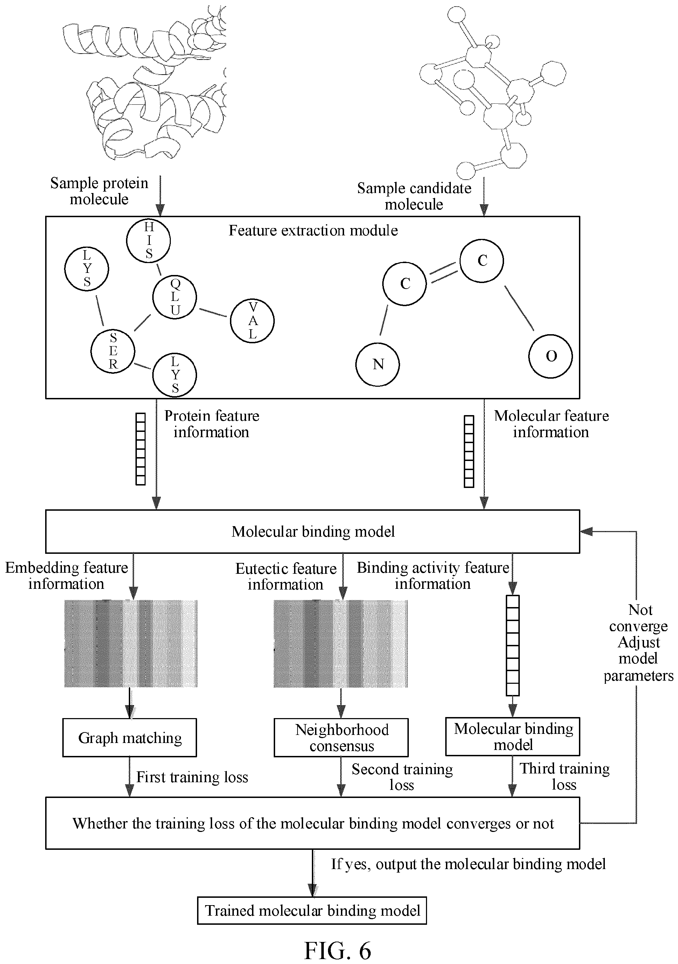

The method for training molecular binding model provided in the embodiment of this application is described below, and please refer to FIG. 6.

The server obtains the sample protein molecule and the sample candidate molecule, uses the feature extraction model to determine the adjacency matrix of the sample protein molecule and the adjacency matrix of the sample candidate molecule respectively, and uses the feature extraction model to perform feature extraction processing on the adjacency matrix of the sample protein molecule and the adjacency matrix of the sample candidate molecule respectively to obtain the protein feature information and the molecular feature information.

The server uses the molecular binding model to determine, based on the protein feature information and the molecular feature information, the binding activity feature information, the embedding feature information and the eutectic feature information between the sample protein molecule and the sample candidate molecule, based on graph matching, obtains the first error value between the embedding feature information and the embedding target, and based on neighborhood consensus, obtains the second error value between the eutectic feature information and the eutectic target. The server uses the activity prediction model to obtain, based on the binding activity feature information, a sample activity value, by training sample, obtains a reference activity value and determines the third error value between the sample activity value and the reference activity value.

The server determines the first training loss of the molecular binding model based on the first error value, determines the second training loss of the molecular binding model based on the second error value, and the third training loss of the molecular binding model based on the third error value.

When determining that there is a non-converging training loss in the various training losses, the server determines that the training loss of the molecular binding model does not converge and the parameters of the molecular binding model are adjusted. When determining that the training losses all converge, the server determines that the training loss of the molecular binding model converges, the molecular binding model is output, and the trained molecular binding model is obtained.

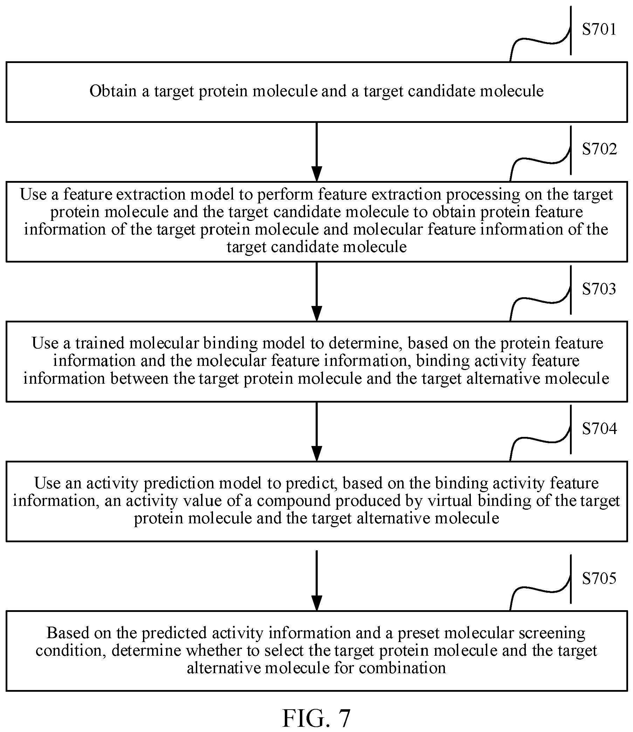

An embodiment of this application provides a method for screening molecules, and the trained molecular binding model trained and obtained by the method for training the molecular binding model described previously is used in performing molecular screening; please refer to FIG. 7, which is a schematic flowchart of the method for screening molecules.

S701: Obtain a target protein molecule and a target candidate molecule.

In performing molecular screening, the target protein molecule and the target candidate molecule may be obtained first. There are various methods for obtaining the target protein molecule and the target candidate molecule, and as an embodiment, a target client may obtain the target protein molecule and the target candidate molecule in response to a molecular selection operation triggered by a target object. The target client sends the target protein molecule and the target candidate molecule to a server, and the server may receive the target protein molecule and the target candidate molecule sent by the target client.

As an embodiment, a protein molecule set and a candidate molecule set may be preset in the server, and the server may select a protein molecule in the protein molecule set as the target protein molecule and select a candidate molecule in the candidate molecule set as the target candidate molecule when performing molecular screening. The server, when performing selection in the protein molecule set or the candidate molecule set, may perform selection randomly or may, based on the attribute information of molecules contained in the set, perform selection according to a preset selection strategy, etc., without specific limitation.

S702: Use a feature extraction model to perform feature extraction processing on the target protein molecule and the target candidate molecule to obtain protein feature information of the target protein molecule and molecular feature information of the target candidate molecule.

S703: Use a trained molecular binding model to determine, based on the protein feature information and the molecular feature information, binding activity feature information between the target protein molecule and the target candidate molecule.

The trained molecular binding model may be trained by other devices and then sent to the server, and the server receives the trained molecular binding model sent by the other devices, or the trained molecular binding model may be a trained molecular binding model obtained by the server from the molecular binding model, without specific limitation.

S704: Use an activity prediction model to predict, based on the binding activity feature information, an activity value of the target protein molecule and the target candidate molecule after virtual binding.

After obtaining a virtual target compound, and virtual target embedding information and virtual target eutectic information corresponding to the virtual target compound, the server uses a target molecular structure model to obtain, based on the virtual target compound, the virtual target embedding information and the virtual target eutectic information, predicted activity information corresponding to the virtual target compound. The process is similar to the previously described process of using the molecular binding model to obtain, based on a sample compound, sample embedding information and sample eutectic information, sample activity information corresponding to the sample compound, and is not repeated here.

S705: Based on predicted activity information and a preset molecular screening condition, determine whether to select the target protein molecule and the target candidate molecule for binding.

After obtaining the predicted activity information, the server may determine, based on the predicted activity information and the preset molecular screening condition, whether to select the target protein molecule and the target candidate molecule for binding. The preset molecular screening condition may be a preset activity threshold, and the target protein molecule and the target candidate molecule are selected for binding in response to that the predicted activity value is greater than or equal to the preset activity threshold. In response to that the predicted activity value is less than the preset activity threshold, the target protein molecule and the target candidate molecule are not selected for binding.

The preset molecular screening condition may also be a preset ranking number, and after obtaining the predicted activity information of each group of target protein molecule and target candidate molecule, activity values contained in each predicted activity information are ranked in a descending order, and the target protein molecule and the target candidate molecule ranked before the preset ranking number are selected for binding.

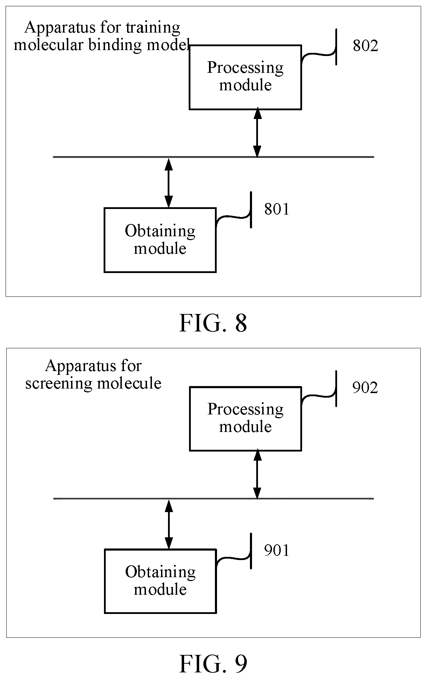

An embodiment of this application provides an apparatus for training molecular binding model, and the apparatus may be a server as previously described, capable of achieving the functions corresponding to the previously described method for training molecular binding model. Please refer to FIG. 8, and the apparatus includes an obtaining module 801 and a processing module 802,
the obtaining module 801, configured to obtain protein feature information of a sample protein molecule and molecular feature information of a sample candidate molecule; and
the processing module 802, configured to input the protein feature information and the molecular feature information into a molecular binding model, and use the molecular binding model to determine binding activity feature information, embedding feature information and eutectic feature information between the sample protein molecule and the sample candidate molecule, the binding activity feature information characterizing an activity of the sample protein molecule and the sample candidate molecule after virtual binding, the embedding feature information characterizing a degree of binding between the sample protein molecule and the sample candidate molecule, and the eutectic feature information characterizing whether a eutectic structure exists between the sample protein molecule and the sample candidate molecule;
determine a training loss of the molecular binding model based on the binding activity feature information, the embedding feature information and the eutectic feature information; and
output the molecular binding model as a trained molecular binding model in response to that the training loss of the molecular binding model meets a training target; the trained molecular binding model being configured to determine binding activity feature information between a target protein molecule and a target candidate molecule to predict the binding activity of a compound after virtual binding of the target protein molecule and the target candidate molecule.

In an exemplary embodiment, the obtaining module 801 is specifically configured to:
obtain training samples, the training sample including the sample protein molecule and the sample candidate molecule; and
use a feature extraction model to perform feature extraction processing on the training sample to obtain protein feature information of the sample protein molecule and molecular feature information of the sample candidate molecule.

In an exemplary embodiment, the obtaining module 801 is specifically configured to:
determine an adjacency matrix of the sample protein molecule based on the sample protein molecule in the training sample, the adjacency matrix of the sample protein molecule being used for characterizing various sample amino acid molecules contained in the sample protein molecule and the molecular structure distance between every two sample amino acid molecules;
determine an adjacency matrix of the sample candidate molecule based on the sample candidate molecule in the training sample, the adjacency matrix of the sample candidate molecule being used for characterizing various sample candidate atoms contained in the sample candidate molecule, and chemical bond structures between the various sample candidate atoms; and
perform feature extraction processing on the adjacency matrix of the sample protein molecule and the adjacency matrix of the sample candidate molecule respectively to obtain the protein feature information of the sample protein molecule, and the molecular feature information of the sample candidate molecule.

In an exemplary embodiment, the processing module 802 is specifically configured to:
predict for each sample amino acid molecule contained in the sample protein molecule, based on the protein feature information and the molecular feature information, the binding distance between the sample amino acid molecule and each sample candidate atom contained in the sample candidate molecule after virtual binding of the sample protein molecule and the sample candidate molecule so as to obtain a plurality of binding distances; and
determine, based on the plurality of binding distances obtained, the embedding feature information and the eutectic feature information between the sample protein molecule and the sample candidate molecule.

In an exemplary embodiment, the processing module 802 is specifically configured to:
determine, based on the binding distance with a minimum value between a specified sample amino acid molecule and each sample candidate atom, the embedding feature information between the sample protein molecule and the sample candidate molecule; the specified sample amino acid molecule being one of the various sample amino acid molecules contained in the sample protein molecule; and
determine, based on the sample amino acid molecule, the sample candidate atom, and a binding distance between the sample amino acid molecule and the sample candidate atom, the eutectic feature information between the sample protein molecule and the sample candidate molecule.

In an exemplary embodiment, the processing module 802 is specifically configured to:
determine, based on a first error value between the embedding feature information and an embedding target, a first training loss of the molecular binding model;
determine, based on a second error value between the eutectic feature information and a eutectic target, a second training loss of the molecular binding model;
determine, based on a third error value between the binding activity feature information and an activity target, a third training loss of the molecular binding model; and
determine, based on the first training loss, the second training loss and the third training loss which are obtained, the training loss of the molecular binding model.

In all embodiments of the present disclosure, the processing module 802 can be specifically configured to:
use the trained neighborhood consensus model to match each sample amino acid molecule contained in the sample protein molecule with each sample candidate atom contained in the sample candidate molecule to obtain matching feature information between the sample protein molecule and the sample candidate molecule as the eutectic target, the matching feature information being used for characterizing the matching distance between each sample amino acid molecule and each sample candidate atom;
use a cross entropy function to determine the second error value between the eutectic feature information and the eutectic target; and
use the second error value as the second training loss of the molecular binding model.

In an exemplary embodiment, the obtaining module 801 is further configured to:
obtain training samples, the training sample including the sample protein molecule, the sample candidate molecule and a reference activity value of the sample protein molecule and the sample candidate molecule after virtual binding; and
the processing module 802 is specifically configured to:
   use an activity prediction model to predict, based on the binding activity feature information, a sample activity value of the sample protein molecule and the sample candidate molecule after virtual binding;
   determine, based on a third error value between the sample activity value and the corresponding reference activity value, the third training loss of the molecular binding model.

In an exemplary embodiment, the processing module 802 is further configured to:
adjust model parameters of the molecular binding model in response to determining that the training loss of the molecular binding model does not meet the training target.

In an exemplary embodiment, the processing module 802 is specifically configured to:
when the training losses obtained include a first training loss, a second training loss and a third training loss, the first training loss being determined based on the embedding feature information, the second training loss being determined based on the eutectic feature information and the third training loss being determined based on the binding activity feature information, determine whether the first training loss converges, whether the second training loss converges, and whether the third training loss converges, respectively; and
adjust model parameters of the molecular binding model in response to that in the first training loss, the second training loss and the third training loss, there is at least one training loss that does not converge.

An embodiment of this application provides an apparatus for screening molecules, and the apparatus may be a server as previously described, capable of achieving the functions corresponding to the previously described method for screening molecules. Please refer to FIG. 9, and the apparatus includes an obtaining module 901 and a processing module 902,
the obtaining module 901, configured to obtain a target protein molecule and a target candidate molecule;
the processing module 902, configured to use a feature extraction model to perform feature extraction processing on the target protein molecule and the target candidate molecule to obtain protein feature information of the target protein molecule and molecular feature information of the target candidate molecule;
the processing module 902 is further configured to: use a trained molecular binding model to determine, based on the protein feature information and the molecular feature information, binding activity feature information between the target protein molecule and the target candidate molecule; the trained molecular binding model being trained and obtained based on the method for training molecular binding model described above;
the processing module 902 is further configured to: use an activity prediction model to predict, based on the binding activity feature information, an activity value of the target protein molecule and the target candidate molecule after virtual binding.

An embodiment of this application provides a computer device, the computer device 1000 being described below.

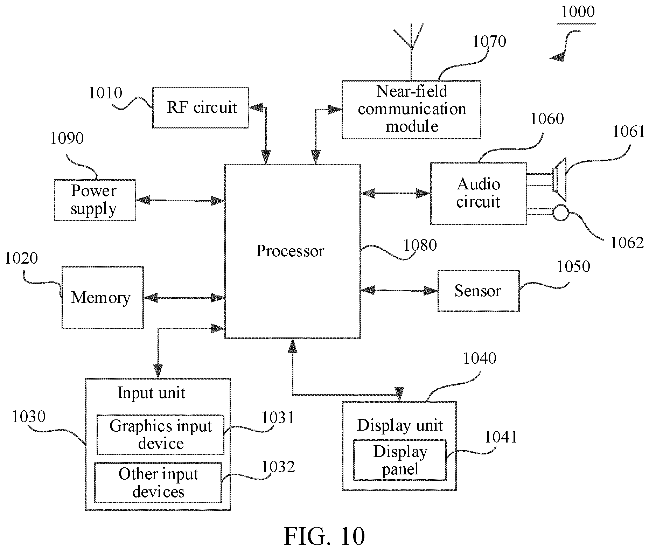

Please refer to FIG. 10, the above described apparatus for training molecular binding model or the apparatus for screening molecules may run on the computer device 1000, and current and historical versions of a data storage program and application software corresponding to the data storage program may be installed on the computer device 1000, and the computer device 1000 includes a display unit 1040, a processor 1080, and a memory 1020, the display unit 1040 including a display panel 1041 for displaying an interface for interactive operation by a user, etc.

In one possible embodiment, the display panel 1041 may be configured in the form of a Liquid Crystal Display (LCD) or an Organic Light-Emitting Diode (OLED), etc.

The processor 1080 is configured to read a computer program and then execute a method defined by the computer program, for example, the processor 1080 reads a data storage program or file, etc., so as to run the data storage program on the computer device 1000 and display a corresponding interface on the display unit 1040. The processor 1080 may include one or more general purpose processors and may also include one or more Digital Signal Processors (DSP) for performing the relevant operations to implement the technical solutions provided by the embodiments of this application.

The memory 1020 generally includes an internal memory and an external memory, the internal memory may be a random access memory (RAM), a read-only memory (ROM), a highspeed cache (CACHE), etc. The external memory can be a hard disk, an optical disk, a USB disk, a floppy disk or a magnetic tape unit, etc. The memory 1020 is configured to store computer programs and other data, the computer program including applications corresponding to each client, etc., the other data including data generated after an operating system or application has been run, the data including system data (e.g., configuration parameters of the operating system) and user data. Program instructions in the embodiment of this application are stored in the memory 1020 and the processor 1080 executes the program instruction stored in the memory 1020 to implement any one of the method for training molecular binding model or the method for screening molecules as described in the preceding figures.

The display unit 1040 described above is configured to receive input digital information, character information or contact touch operations/non-contact gestures, and generate signal input related to user settings and function control, etc. of the computer device 1000. Specifically, in the embodiment of this application, the display unit 1040 may include a display panel 1041. The display panel 1041, such as a touch screen, may collect a touch operation of a user on or near the display panel (such as an operation of a user on the display panel 1041 or near the display panel 1041 by using any suitable object or accessory such as a finger or a stylus), and drive a corresponding connection apparatus according to a preset program.

In a possible embodiment, the display panel 1041 may include two parts: a touch detection apparatus and a touch controller. The touch detection apparatus detects a touch location of a player, detects a signal generated by a touch operation, and transmits the signal to the touch controller. The touch controller receives touch information from the touch detection apparatus, converts the touch information into touch point coordinates, and then sends the touch point coordinates to the processor 1080, and can receive and execute a command sent from the processor 1080.

The display panel 1041 may be implemented as display panels in various types of resistive, capacitive, infrared, or surface sound wave types. In addition to the display unit 1040, the computer device 1000 may also include an input unit 1030, the input unit 1030 may include a graphics input device 1031 and other input devices 1032, where the other input devices may include, but are not limited to, one or more of a physical keyboard, function keys (such as volume control buttons, switch buttons, etc.), a trackball, a mouse, an operating stick, and the like.

In addition to the above, the computer device 1000 may include a power supply 1090 for supplying power for other modules, an audio circuit 1060, a near-field communication module 1070 and an RF circuit 109. The computer device 1000 may also include one or more sensors 1050, such as an acceleration sensor, a photo sensor, a pressure sensor, etc. The audio circuit 1060 specifically includes a loudspeaker 1061 and a microphone 1062, etc. For example, the computer device 1000 may collect the user's voice through the microphone 1062 to perform corresponding operations, etc.

As an embodiment, the number of the processor 1080 may be one or more, and the processor 1080 and the memory 1020 may be coupled or relatively independent.

As an embodiment, the processor 1080 in FIG. 10 may be configured to implement the functions of the obtaining module 801 and the processing module 802 as in FIG. 8, or may be configured to implement the functions of the obtaining module 901 and the processing module 902 as in FIG. 9.

As an embodiment, the processor 1080 in FIG. 10 may be configured to implement the functions corresponding to the server 102 as described previously.

A person of ordinary skill in the art may understand that all or some steps for implementing the foregoing method embodiment may be completed by hardware related to a program instruction, the foregoing program may be stored in a computer-readable storage medium, and when being executed, the program performs steps including the foregoing method embodiment. The foregoing storage medium includes any medium that can store program codes, such as a removable storage device, a read-only memory (ROM), a random access memory (RAM), a magnetic disk, or an optical disc, etc.

Alternatively, when the above integrated unit in this application is implemented in the form of a software function module and sold or used as an independent product, the integrated unit may also be stored in a computer-readable storage medium. Based on such an understanding, the technical solutions of the embodiments of this application essentially or the part contributing to the related art may be embodied in the form of a software product. The computer software product is stored in a storage medium, and includes several instructions used to cause a computer device (which may be a personal computer, a server, or a network device) to execute all or a part of the method described in the embodiments of this application. The foregoing storage medium includes any medium that can store program codes, such as a removable storage device, a ROM, a RAM, a magnetic disk, or an optical disc, etc.

Obviously, a person skilled in the art can make various modifications and variations to this application without departing from the spirit and scope of this application. In this case, if the modifications and variations made to this application fall within the scope of the claims of this application and their equivalent technologies, this application is intended to include these modifications and variations.

## Claims

1. A method for training a molecular binding model, executable by a computer device, comprising:
obtaining protein feature information of a sample protein molecule and molecular feature information of a sample candidate molecule;
inputting the protein feature information and the molecular feature information into a molecular binding model, and determining, using the molecular binding model, binding activity feature information, embedding feature information and eutectic feature information between the sample protein molecule and the sample candidate molecule, the binding activity feature information characterizing an activity of a compound produced by virtual binding of the sample protein molecule and the sample candidate molecule, the embedding feature information characterizing a degree of binding between the sample protein molecule and the sample candidate molecule, and the eutectic feature information characterizing whether an eutectic structure exists between the sample protein molecule and the sample candidate molecule;
determining a training loss of the molecular binding model based on the binding activity feature information, the embedding feature information and the eutectic feature information; and
outputting the molecular binding model as a trained molecular binding model in response to the training loss of the molecular binding model meets a training target; the trained molecular binding model being configured to determine binding activity feature information between a target protein molecule and a target candidate molecule to predict the binding activity of a compound produced by virtual binding of the target protein molecule and the target candidate molecule.

2. The method according to claim 1, wherein obtaining the protein feature information of the sample protein molecule and the molecular feature information of the sample candidate molecule, comprises:
obtaining a training sample, the training sample comprising the sample protein molecule and the sample candidate molecule; and
extracting, using a feature extraction model, the protein feature information of the sample protein molecule and the molecular feature information of the sample candidate molecule.

3. The method according to claim 2, wherein extracting, using the feature extraction model, the protein feature information of the sample protein molecule and the molecular feature information of the sample candidate molecule, comprises:
determining an adjacency matrix of the sample protein molecule, the adjacency matrix of the sample protein molecule characterizing sample amino acid molecules contained in the sample protein molecule and a molecular structure distance between every two sample amino acid molecules;
determining an adjacency matrix of the sample candidate molecule, the adjacency matrix of the sample candidate molecule characterizing sample candidate atoms contained in the sample candidate molecule and chemical bond structures between the sample candidate atoms; and
extracting features of the adjacency matrix of the sample protein molecule and the adjacency matrix of the sample candidate molecule respectively to obtain the protein feature information of the sample protein molecule and the molecular feature information of the sample candidate molecule.

4. The method according to claim 1, wherein inputting the protein feature information and the molecular feature information into the molecular binding model, and determining, using the molecular binding model, the embedding feature information and the eutectic feature information between the sample protein molecule and the sample candidate molecule, comprises:
predicting for each of sample amino acid molecules contained in the sample protein molecule, based on the protein feature information and the molecular feature information and using the molecular binding model, a binding distance between the sample amino acid molecule and each of sample candidate atoms contained in the sample candidate molecule to obtain a plurality of binding distances; and
determining, based on the plurality of binding distances, the embedding feature information and the eutectic feature information between the sample protein molecule and the sample candidate molecule.

5. The method according to claim 4, wherein determining, based on the plurality of binding distances, the embedding feature information and the eutectic feature information between the sample protein molecule and the sample candidate molecule comprises:
determining, based on a binding distance with a minimum value among binding distances between a specified sample amino acid molecule and each of sample candidate atoms, the embedding feature information between the sample protein molecule and the sample candidate molecule; the specified sample amino acid molecule being one of the sample amino acid molecules contained in the sample protein molecule; and
determining, based on the sample amino acid molecules, the sample candidate atoms, and the binding distances between the sample amino acid molecules and the sample candidate atoms, the eutectic feature information between the sample protein molecule and the sample candidate molecule.

6. The method according to claim 1, wherein determining the training loss of the molecular binding model based on the binding activity feature information, the embedding feature information and the eutectic feature information, comprises:
determining, based on a first error value between the embedding feature information and an embedding target, a first training loss of the molecular binding model;
determining, based on a second error value between the eutectic feature information and a eutectic target, a second training loss of the molecular binding model;
determining, based on a third error value between the binding activity feature information and an activity target, a third training loss of the molecular binding model; and
determining, based on the first training loss, the second training loss and the third training loss, the training loss of the molecular binding model.

7. The method according to claim 6, wherein determining, based on the second error value between the eutectic feature information and the eutectic target, the second training loss of the molecular binding model, comprises;
matching, using a trained neighborhood consensus model, sample amino acid molecules contained in the sample protein molecule with sample candidate atoms contained in the sample candidate molecule to obtain matching feature information between the sample protein molecule and the sample candidate molecule, and determining the matching feature information as the eutectic target, the matching feature information characterizing matching distances between the sample amino acid molecules and the sample candidate atoms;
determining, using a cross entropy function, the second error value between the eutectic feature information and the eutectic target; and
using the second error value as the second training loss of the molecular binding model.

8. The method according to claim 6, further comprising:
obtaining a reference activity value for the compound produced by virtual binding of the sample protein molecule and the sample candidate molecule; and
determining, based on the third error value between the binding activity feature information and the activity target, the third training loss of the molecular binding model, comprises:
predict, using an activity prediction model and based on the binding activity feature information, a sample activity value of the compound produced by virtual binding of the sample protein molecule and the sample candidate molecule; and
determining, based on the third error value between the sample activity value and the reference activity value, the third training loss of the molecular binding model.

9. The method according to claim 1, after determining the training loss of the molecular binding model based on the binding activity feature information, the embedding feature information and the eutectic feature information, further comprising:
adjusting model parameters of the molecular binding model in response to determining that the training loss of the molecular binding model does not meet the training target.

10. The method according to claim 9, wherein adjusting the model parameters of the molecular binding model in response to determining that the training loss of the molecular binding model does not meet the training target, comprises:
in response to that the training losses comprise a first training loss, a second training loss and a third training loss, the first training loss being determined based on the embedding feature information, the second training loss being determined based on the eutectic feature information and the third training loss being determined based on the binding activity feature information, determining whether the first training loss converges, whether the second training loss converges, and whether the third training loss converges, respectively; and
adjusting the model parameters of the molecular binding model in response to that at least one of the first training loss, the second training loss and the third training loss does not converge.

11. A method for screening molecules, executable by a computer device, the method comprising:
obtaining a target protein molecule and a target candidate molecule;
extracting, using a feature extraction model, protein feature information of the target protein molecule and molecular feature information of the target candidate molecule;
determining, using a molecular binding model and based on the protein feature information and the molecular feature information, binding activity feature information between the target protein molecule and the target candidate molecule; the molecular binding model being trained and obtained based on the method according to any one of claims 1 to 10; and
predicting, using an activity prediction model and based on the binding activity feature information, an activity value of a compound produced by virtual binding of the target protein molecule and the target candidate molecule.

12. An apparatus for training a molecular binding model, comprising:
an obtaining module, configured to obtain protein feature information of a sample protein molecule and molecular feature information of a sample candidate molecule; and
a processing module, configured to input the protein feature information and the molecular feature information into a molecular binding model, and determine, using the molecular binding model, binding activity feature information, embedding feature information and eutectic feature information between the sample protein molecule and the sample candidate molecule, the binding activity feature information characterizing an activity of a compound produced by virtual binding of the sample protein molecule and the sample candidate molecule, the embedding feature information characterizing a degree of binding between the sample protein molecule and the sample candidate molecule, and the eutectic feature information characterizing whether a eutectic structure exists between the sample protein molecule and the sample candidate molecule;
determine a training loss of the molecular binding model based on the binding activity feature information, the embedding feature information and the eutectic feature information; and
output the molecular binding model as a trained molecular binding model in response to that the training loss of the molecular binding model meets a training target; the trained molecular binding model being configured to determine binding activity feature information between a target protein molecule and a target candidate molecule to predict the binding activity of the compound produced by virtual binding of the target protein molecule and the target candidate molecule.

13. An apparatus for screening molecules, comprising:
an obtaining module, configured to obtain a target protein molecule and a target candidate molecule;
a processing module, configured to extract, using a feature extraction model, protein feature information of the target protein molecule and molecular feature information of the target candidate molecule;
the processing module being further configured to: determine, using a molecular binding model and based on the protein feature information and the molecular feature information, binding activity feature information between the target protein molecule and the target candidate molecule; the molecular binding model being trained and obtained based on the method according to any one of claims 1 to 10; and
the processing module being further configured to: predict, using an activity prediction model and based on the binding activity feature information, an activity value of a compound produced by virtual binding of the target protein molecule and the target candidate molecule.

14. A computer device, comprising:
a memory, configured to store program instructions; and
a processor, configured to call program instructions stored in the memory, to perform, in accordance with the obtained program instruction, the method according to any one of claims 1 to 11.

15. A computer-readable storage medium, the storage medium storing computer-executable instructions, and the computer-executable instruction being configured to cause a computer to perform the method according to any one of claims 1 to 11.

16. A computer program product, the computer program product comprising computer instructions, the computer instructions being stored in a computer-readable storage medium, and the computer instructions, when being executed by the computer instructions, implementing the method according to any one of claims 1 to 11.
